# EUROPÄISCHE PATENTSCHRIFT

(11) **EP 1 400 554 B1**
(45) Veröffentlichungstag und Bekanntmachung des Hinweises auf die Patenterteilung: **01.03.2006**
(21) Anmeldenummer: 03020215.4
(22) Anmeldetag: 06.09.2003
(51) Int. Cl.: C08G 77/46, C08G 77/445, B01F 17/00

(54) **Polyestergruppen aufweisende amphiphile Organopolysiloxane und deren Verwendung als Emulgatoren oder Dispergierhilfsmittel**
Polyester group containing amphiphilic organopolysiloxanes and their use as emulsifying or dispersing agents
Organopolysiloxanes amphiphiles contenant des groupes polyester et leur application comme agents émulsifiants ou dispersants

(30) Priorität: 21.09.2002 DE 10243992
(43) Veröffentlichungstag der Anmeldung: 24.03.2004
(73) Patentinhaber: Goldschmidt GmbH, 45127 Essen (DE)
(72) Erfinder: Dietz, Thomas, Dr., Glen Allen VA 23060 (US); Oestreich, Sascha, Dr., 45279 Essen (DE)

(56) Entgegenhaltungen:
- EP-A- 0 819 426
- EP-B- 0 608 921
- EP-B- 0 879 840
- US-A- 5 411 729
- US-A- 5 475 125
- US-A- 5 929 268

## Beschreibung

Gegenstand der Erfindung sind Polyestergruppen aufweisende amphiphile Organopolysiloxane und deren Verwendung als Emulgatoren oder als Dispergierhilfsmittel.

Polyestergruppen aufweisende Organopolysiloxane sind seit langem bekannt. So beschreibt beispielsweise die US-A-5 051 489 hydrophobe Siliconpolyesterwachse, die durch Veresterung der Silanolgruppen von Siloxandiolen mit Dicarbonsäuren und Fettsäuren erhalten werden. Hierbei handelt es sich um blockweise aus Dialkoholen und Dicarbonsäuren aufgebaute Polyester, bei denen das Polysiloxan die Funktion des Dialkohols übernimmt.

In der US-A-5 385 730 werden Mischungen beansprucht, bestehend aus einem niedrigviskosen Siliconöl und einem blockweise aufgebauten Polyesterrest tragenden Polysiloxan, dessen Polyesterreste durch Lactonringöffnung erhalten werden. Neben den Polyesterresten können diese Polysiloxane auch langkettige Alkylreste tragen. Es handelt sich hierbei um hydrophobe Polysiloxane, die als Glanzmittel verwendet werden können.

Die JP-B-3046340 beschreibt Polyester, die durch Reaktion von terminalen aliphatischen Hydroxylgruppen enthaltenden Siloxandiolen und Dicarbonsäuren, wie Adipinsäure und anschließender Umsetzung mit Monocarbonsäuren, z. B. auch Hydroxystearinsäure, erhalten werden. Die Siliconpolyester werden zur Verwendung in der Kosmetik empfohlen.

In der US-A-5 411 729 werden unter anderem Siliconpolyester beschrieben, die durch Umsetzung von seitenständigen oder terminalen Polyetherresten mit freien OH-Gruppen tragenden Siloxanen mit Dicarbonsäuren und Polyhydroxyverbindungen, wie Glycerin und gegebenenfalls mit einer monofunktionellen Carbonsäure erhalten werden. Die Verbindungen werden als Konditioniermittel für das Haar beansprucht.

Die US-A-5 475 125 beansprucht amphiphile Siliconpolyester, die durch Reaktion eines kammartigen Polyethersiloxans mit freien OH-Gruppen mit einer Dicarbonsäure und einem Fettalkoholethoxylat erhalten werden. Sie werden als Emulgatoren für Siliconölin-Wasser-Emulsionen empfohlen.

Aus der Beschreibung des Standes der Technik wird ersichtlich, dass Siliconpolyester unterschiedlicher Struktur bekannt sind.

Man kann zwei Grundtypen von Polyestern unterscheiden. Zum einen solche, die durch Polykondensation von Polyalkoholen mit Dicarbonsäuren erhalten werden und solche, die durch Selbstkondensation von hydroxyfunktionellen Carbonsäuren bzw. durch Ringöffnungsreaktion entsprechender Lactone erhalten werden.

Der erstgenannte Typ ist blockweise aufgebaut, wenn der Polyalkohol ein Diol ist. Hierbei kann das Polysiloxan die Funktion des Diols übernehmen wie in der US-A-5 051 489 oder der JP-B-3046340 beschrieben. Das Siloxandiol kann zum einen silanolartige OH-Gruppen tragen, was zu leicht hydrolysierbaren Si-O-C-Bindungen führt, oder Hydroxyalkyl-Gruppen, die hydrolyseresistente Si-C-Bindungen ergeben.

Das Siloxan kann jedoch auch mehr als 2 OH-Gruppen tragen wie in der US-A-5 475 125 dargelegt. Nachteil der so hergestellten Siliconpolyester ist, dass die eingesetzte Dicarbonsäure verbrückend zwischen zwei Siliconpolyethern wirken kann und schwer kontrollierbare Vernetzungsreaktionen auslöst, die zur völligen vergelung des Produktes führen können.

Aus der in US-A-5 411 729 sind auch Siliconpolyester bekannt, die neben dem polyhydroxyfunktionellen Siloxan zusätzlich eine organische Polyhydroxyverbindung als Baustein enthalten. Aber auch in diesem Fall ist mit Vernetzungreaktionen zu rechnen.

Kontrollierter herstellen lassen sich Siliconpolyester durch Ringöffnungsreaktionen von Lactonen mit hydroxyfunktionellen Siloxanen (US-A-5 385 730). Auf diese Weise können Vernetzungreaktionen vollständig vermieden werden.

Grundsätzlich lassen sich Polyestersiloxane in amphiphile und nicht amphiphile Typen einteilen. Siloxane, die nur Polyesterreste tragen sind in der Regel hydrophob und zeigen keine Grenzflächenaktivität zwischen einer hydrophilen und einer hydrophoben Phase. Polyestersiloxane, die zusätzlich hydrophile Gruppen wie z. B. Polyetherreste tragen (US-A-5 411 729 und US-A-5 475 125) sind hingegen grenzflächenaktiv und können zum Beispiel als Emulgatoren eingesetzt werden.

Die Verwendung von amphiphil modifizierten Polysiloxanen, insbesondere polyethermodifizierten Polysiloxanen, als Emulgatoren und Dispergierhilfsmittel ist seit langem bekannt. So ist die Verwendung von grenzflächenaktiven Siliconpolyethern in Emulgatorsystemen beispielsweise in US-A-4 988 504 ausführlich beschrieben. Es werden Polysiloxanverbindungen vorgeschlagen, die aus
a) Einheiten der Formel

   R₂SiO_{2/2}

   - R =: Wasserstoff oder ein substituierter oder unsubstituierter Kohlenwasserstoffrest mit 1 bis 12 Kohlenstoffatomen,
b) Einheiten der Formel

   RR¹SiO_{2/2}

   - R¹ =: Polyalkylenether der Formel

   -R³ₐ-(OR²)ₙ-OR⁴

   - R² =: -CH₂CH₂-,
   - R³ =: substituierte oder unsubstituierte Alkylengruppe mit 1 bis 20 Kohlenstoffatomen,
   - R⁴ =: R,
   - n: hat einen Wert von 5 bis 20 und
   - a: ist 0 oder 1, und
c) Endgliedern der Siloxankette
bestehen, wobei das Molekulargewicht der Komponente a) etwa 25.000 bis 35.000 betragen soll.
Diese Emulgatoren mit seitenständigen Polyoxyalkylengruppen sollen verbesserte Eigenschaften für Antiperspirantsticks in bezug auf Stabilität und niedrigen Wachsanteil bewirken.

Die EP-B-O 407 089 betrifft eine transparente Wasser-in-Silikonöl-Emulsion, geeignet zur äußeren Anwendung auf Säugetierhaut oder -haar, umfassend zusätzlich zu Wasser:
i. 1 bis 50 Gew.-% eines flüchtigen Polydimethylsiloxans;
ii. 0,1 bis 20 Gew.-% eines Silikontensid-Inhaltsstoffs, umfassend ein Polymer von Dimethylpolysiloxan mit Polyoxyethylen- und/oder Polyoxypropylen-Seitenketten mit einem Molekulargewicht von 10.000 bis 50.000 und der Strukturformel: 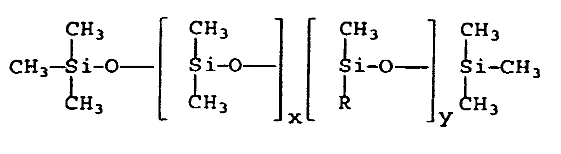 worin
   - R =: -H oder 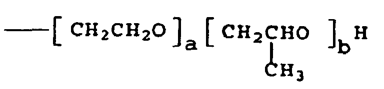
   ist,
   - a: einen Wert von 9 bis 115 hat,
   - b: einen Wert von 0 bis 50 hat,
   - x: einen Wert von 133 bis 673 hat,
   - y: einen Wert von 25 bis 0,25 hat und
iii. 1 bis 50 Gew.-% eines transparenzgebenden Mittels, das mindestens ein mehrwertiger Alkohol ist.

In der EP-B-0 176 844 wird die Verwendung von Polysiloxanen mit kammartig angelagerten Polyether- und langkettigen Alkylresten als Emulgatoren zur Herstellung von W/O-Emulsionen, deren ölige Phase aus Siliconöl besteht oder dieses enthält, beansprucht. Die EP-A-0 819 426 beschreibt die Verwendung von endständig modifizierten Polyethersiloxanen als Emulgatoren in W/O-Emulsionen.

Der Nachteil der hier beschriebenen polyethermodifizierten Polysiloxane ist jedoch, dass bei deren Verwendung als Emulgatoren in W/O-Emulsionen unerwünscht hohe Emulsionsviskositäten, insbesondere bei Emulsionen mit einem hohen Wasserphasengehalt, erzielt werden.

Aufgabe der vorliegenden Erfindung ist deshalb die Bereitstellung von Emulgatoren für kosmetische W/O-Emulsionen, die eine niedrige Viskosität aufweisen.

Erfindungsgemäß wird diese Aufgabe gelöst durch die Verwendung von Organopolysiloxan-Copolymeren, enthaltend im Mittel mindestens eine über einen Spacer an das Siloxan gebundene Polyestergruppe und im Mittel mindestens eine über einen Spacer an das Siloxan gebundene hydrophile Gruppe.

Ein Gegenstand der vorliegenden Erfindung sind daher Organopolysiloxan-Copolymere, enthaltend im Mittel mindestens eine über einen Spacer an das Siloxan gebundene Polyestergruppe und im Mittel mindestens eine über einen Spacer an das Siloxan gebundene hydrophile Gruppe, der allgemeinen Formel (I): 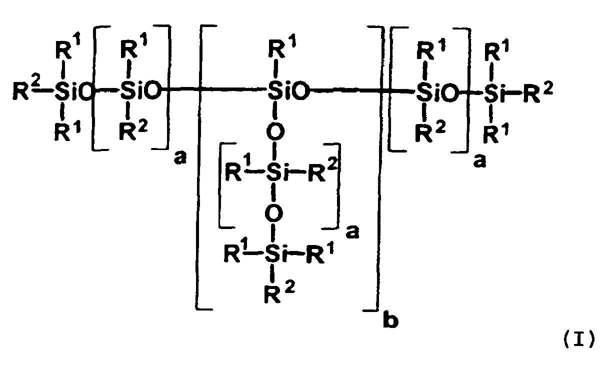
worin bedeuten
- R¹: gleich oder verschieden sind und Alkylreste mit 1 bis 30 Kohlenstoffatomen oder Phenylreste,
- R²: unabhängig voneinander R¹, -A-R³ oder -B-R⁴ in denen
-A- eine divalente Alkylenoxy-Gruppe mit 3 bis 24 C-Atomen, die gegebenenfalls verzweigt ist und/oder Doppelbindungen enthalten kann,
und/oder eine divalente Polyoxyalkylen-Gruppe der allgemeinen durchschnittlichen Formel

-R⁵-(C₂H₄O)_{q}-(C₃H₆O)ᵣ-(C₄H₈O)ₛ-

in der
q = 1 bis 100,
r = 0 bis 100,
s = 0 bis 100,
R⁵ eine divalente Alkylenoxy-Gruppe ist, mit 1 bis 24 C-Atomen, die gegebenenfalls verzweigt ist und/oder Doppelbindungen enthalten kann,
R³ ein Polyesterrest der allgemeinen Formel 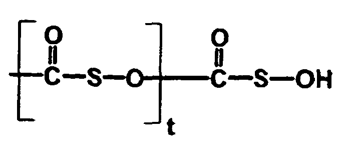 worin
t ganze Zahlen im Bereich von 1 bis 10 darstellt, und [-(O=C)-S-O-] das Fragment einer entsprechenden Hydroxycarbonsäure HO-(O=C)-S- OH bedeutet, in dem
-S- ein gegebenenfalls verzweigter und/oder Doppelbindungen enthaltender Alkylenrest mit 5 bis 30 Kohlenstoffatomen ist, mit der Maßgabe, dass zwischen der Carboxylgruppe [HO-C(O)-] und der Hydroxylgruppe[-OH] mindestens 5 Kohlenstoffatome sind;
-B- als Spacer zwischen Siloxangerüst und dem Rest R⁴ fungiert und von aus dem Stand der Technik für hydrophilmodifizierte Siloxane bekannter Art ist
R⁴ ein hydrophiler Rest der allgemeinen durchschnittlichen Formel

-R⁶-(C₂H₄O)_{q}-(C₃H₆O)ᵣ-(C₄H₈O)ₛ-R⁷

in der
q = 1 bis 100,
r = 0 bis 100,
s = 0 bis 100,
R⁶ eine divalente Alkylen- oder Alkylenoxy-Gruppe mit 1 bis 24 C-Atomen, die gegebenenfalls verzweigt ist und/oder Doppelbindungen enthalten kann;
R⁷ ein Wasserstoffatom, Alkyl- oder Acyl-Rest mit 1 bis 20 C-Atomen ist, oder
R⁴ ein Polyhydroxyorganyl-Rest, insbesondere ein Glycerin-, Polyglycerin-, Zucker- oder Zuckerderivat-Rest, ein Polyvinylalkoholrest, ein Carboxylat-, Sulfat- oder Phosphat-Rest, ein Ammonium-Rest oder ein amphoteres Betain- oder Amphoglycinat-Rest,
- a: einen Wert von 1 bis 1.000, vorzugsweise 5 bis 500, insbesondere 10 bis 150 und
- b: einen Wert von 0 bis 10 hat, insbesondere < 2 ist, mit der Maßgabe, dass im statistischen Mittel mindestens jeweils ein Rest R² = -A-R³ und R² = -B-R⁴ enthalten ist, oder für den Fall, dass kein Rest -B-R⁴ enthalten ist, mindestens ein Rest R² = -A-R³ enthalten ist, bei dem -A- einer divalenten Polyoxyalkylen-Gruppe der oben beschriebenen allgemeinen durchschnittlichen Formel

-R⁵-(C₂H₄O)_{q}-(C₃H₆O)ᵣ-(C₄H₈O)ₛ-

entspricht.

Eine bevorzugte Ausführung des Gegenstands der Erfindung sind Organopolysiloxan-Copolymere der allgemeinen Formel (I), in der das Fragment [-(O=C)-S-O-]ₜ dem Rest der 12-Hydroxystearinsäure oder der Ricinolsäure entspricht und t zwischen 2 und 5 liegt.

Eine weitere bevorzugte Ausführung des Gegenstands der Erfindung sind Organopolysiloxan-Copolymere der allgemeinen Formel (I), in denen der hydrophile Rest ausgewählt ist aus der Gruppe der Polyether.

Ein weiterer Gegenstand der vorliegenden Erfindung ist die Verwendung der Polysiloxan-Copolymeren der allgemeinen Formel (I) als Emulgatoren, gegebenenfalls unter Mitverwendung weiterer Emulgatoren, für die Herstellung niedrigviskoser W/O-Emulsionen mit hohem Gehalt an disperser Phase.

Ein weiterer Gegenstand der Anmeldung sind Verfahren zur Herstellung der erfindungsgemäßen Verbindungen, dadurch gekennzeichntet, dass die Polyesterreste entweder durch Hydrosilylierung von Doppelbindungen tragenden Polyestern an Polyhydrogensiloxane oder durch Veresterung von OH-funktionellen Polysiloxanen mit freien Carboxylgruppen tragenden Polyestern, und die hydrophilen Reste durch aus dem Stand der Technik bekannte Verfahren an das Polysiloxan angelagert werden.

Die Spacer (B) sind die aus dem Stand der Technik bekannten Reste wie C₁₋₂₄ Alkylenrest, welche gegebenenfalls verzweigt sein können, gegebenenfalls Mehrfachbindungen oder Heteroatome wie insbesondere Sauerstoffatome und/oder funktionelle Gruppen wie insbesondere Hydroxylgruppen enthalten können.

Die Verbindungen der allgemeinen Formel (I) werden in Mengen von ca. 0,5 bis ca. 4 Gew.-%, bezogen auf die Gesamtformulierung, zur Herstellung von wässrigen Dispersionen und Emulsionen, insbesondere zur Herstellung von W/O-Emulsionen für kosmetische Zwecke verwendet, wobei die für diese Anwendungsgebiete bekannten Rezepturbestandteile wie Ölkomponente, Fette und Wachse, Polyole, aktive Wirkstoffe wie Vitamine, Fruchtsäuren und Aminosäuren wie Kreatin, Parfümöle, Farbstoffe, Pigmente, UV-Filter, Elektrolyte in den üblichen Mengen mitverwendet werden.

Beispiele erfindungsgemäßer Verbindungen sind:

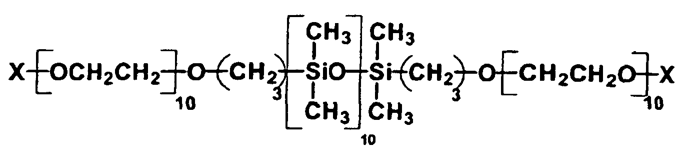

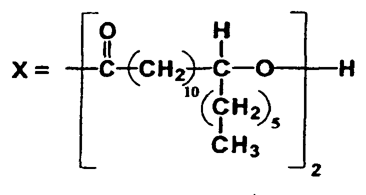

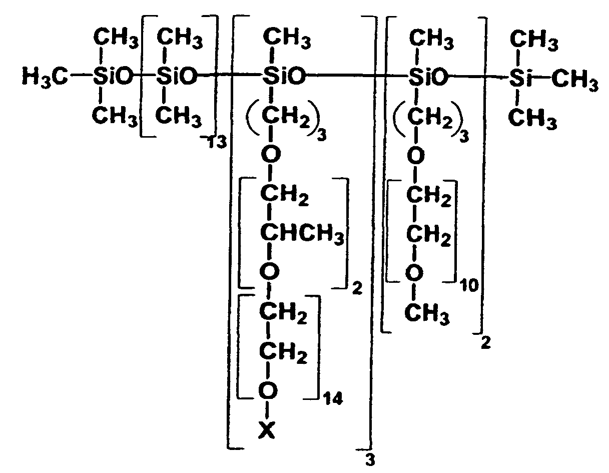

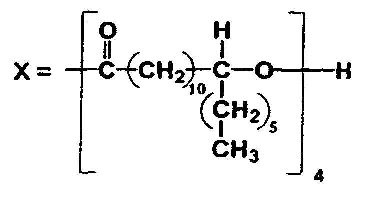

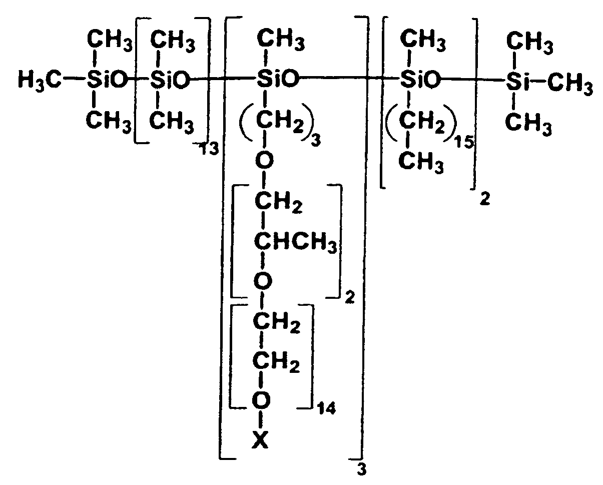

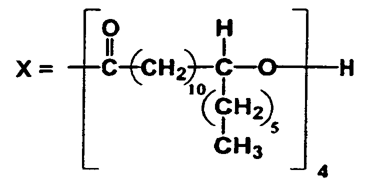

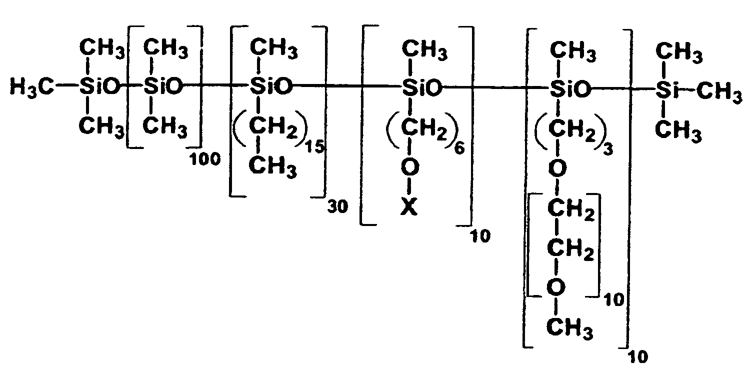

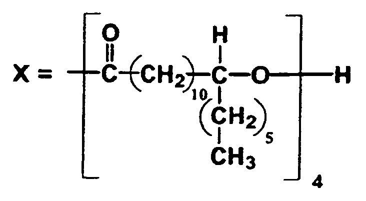

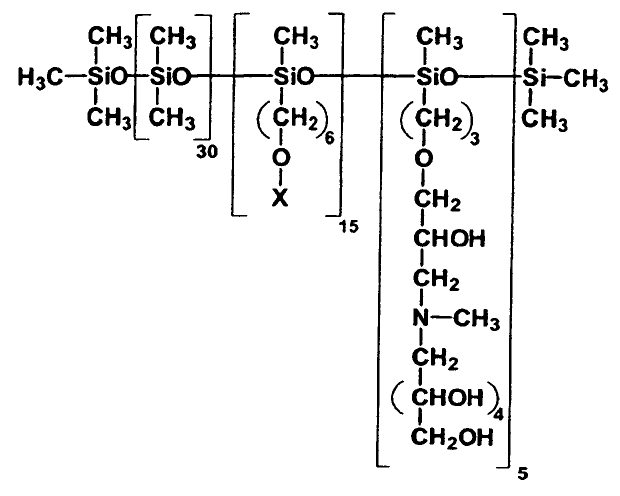

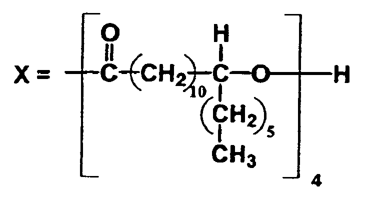

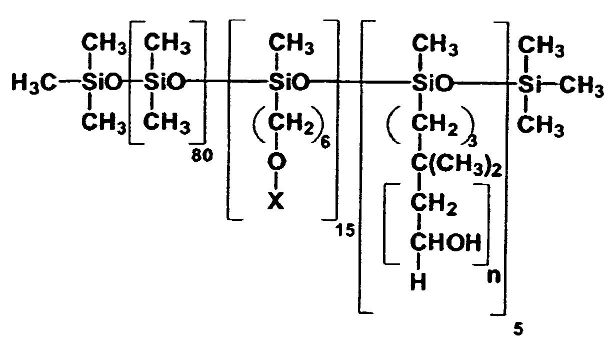

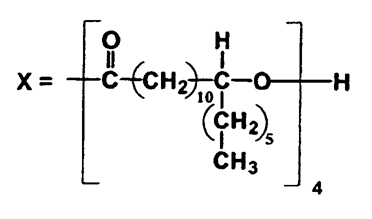

Die folgenden Beispiele veranschaulichen die vorliegende Erfindung.

### Beispiel 1:

71,8 g Polyhydroxystearinsäure (n = 2, Säurezahl = 93,7 mg KOH/g) werden aufgeschmolzen, homogenisiert und zusammen mit 112,2 g eines doppelt endständig modifizierten Polyethersiloxans(Siloxankettenlänge N = 10), Molekulargewicht des Polyethers (100 % Polyethylenglyol) = 600 g/mol, OH-Zahl des Polyethersiloxans: 60 mg KOH/g) und 120 g Toluol in einen 500-ml-Dreihalskolben, ausgestattet mit Wasserabscheider, Rührer und Thermometer, übergeführt. Es werden 0,55 g Methansulfonsäure zugegeben und die Mischung 6 h auf 125 °C unter Rühren erhitzt. Anschließend wird am Rotationsverdampfer das Lösungsmittel bei 80 bis 100 °C entfernt. Man erhält eine klare, bräunlich gefärbte Flüssigkeit. Der Umsatz, bestimmt über den Endwert der Säurezahl, beträgt ca. 99 %.

### Beispiele 2 bis 4:

### Tabelle 1:

Experimentelle Einzelheiten zu den Beispielen 2 bis 4

| Beispiel Nr. | Polyethersiloxan: Siloxankettenlänge N, Molekulargewicht Polyether MW [g/mol], OH-Zahl Polyethersiloxan [mg KOH/g] | Einwaage Polyethersiloxan [g] | Einwaage Polyhydroxystearinsäure [g] | Einwaage Methansulfonsäure [g] | Einwaage Toluol [g] |
|---|---|---|---|---|---|
| 2 | N = 10, | 98,2 | 93,5 | 0,58 | 120 |
| | MW = 400, | | | | |
| | OH-Zahl = 80 | | | | |
| 3 | N = 20, | 114,1 | 80,1 | 0,58 | 120 |
| | MW = 400, | | | | |
| | OH-Zahl = 59 | | | | |
| 4 | N = 80, | 154,3 | 36,7 | 0,57 | 120 |
| | MW = 400, | | | | |
| | OH-Zahl = 93,7 | | | | |

### Anwendungstechnischer Teil:

### Beispiel 5:

| Phase | Emulsion Nr. (Angaben der Inhaltsstoffe in %) | 1 | 2 | 3 | 4 | 5 | 6 | 7 | 8 | 9 |
|---|---|---|---|---|---|---|---|---|---|---|
| A | Emulgator aus Beispiel 2 | - | 2 | - | 1,5 | 1,5 | - | - | - | - |
| | Emulgator aus Beispiel 3 | 2 | - | 2 | - | - | 1,5 | 1,5 | - | - |
| | Cetyl Dimethicone Copolyol (ABIL EM 90) | - | - | - | - | - | - | - | 2 | 2 |
| | Diisostearoyl Polyglyceryl-3 Dimer Dilinoleate (ISOLAN PDI) | - | - | - | 0,5 | 0,5 | 0,5 | 0,5 | - | - |
| | Microcrystalline Wax | - | - | - | - | - | - | 0,25 | - | - |
| | Castor Wax | - | - | - | - | - | - | 0,25 | - | - |
| | Mineral Oil | - | 23 | 23 | 23 | - | - | 8 | 23 | 7,6 |
| | Ethylhexyl Stearate | 23 | - | - | - | 23 | 23 | 8 | - | 7,7 |
| | Caprylic/Capric Triglyceride | - | - | - | - | - | | 8 | - | 7,7 |
| Phase | Emulsion Nr. (Angaben der Inhaltsstoffe in %) | 1 | 2 | 3 | 4 | 5 | 6 | 7 | 8 | 9 |
| B | Glycerin | 2,45 | 2,45 | 2,45 | 2,45 | 2,45 | 2,45 | 3,35 | 2,45 | 2,45 |
| | Bronopol | 0,05 | 0,05 | 0,05 | 0,05 | 0,05 | 0,05 | 0,05 | 0,05 | 0,05 |
| | Magnesium Sulfate Heptahydrate | 0,5 | 0,5 | 0,5 | 0,5 | 0,5 | 0,5 | 0,5 | 0,5 | 0,5 |
| | Water | 72 | 72 | 72 | 72 | 72 | 72 | 68 | 72 | 72 |
| | Emulsions-viskosität [Pas] nach 2 Tagen bei Raumtemperatur (V 1) sowie nach 2 Monaten bei Raumtemperatur (V 2) | V1 = 9 | V1 = 6 | V1 = 10 | V1 = 11 | V1 = 8 | V1 = 8 | V1 = 12 | V1 = 36 | V1 = 52 |
| | | V1 = 7 | V2 = 6 | V2 = 7 | V2 = 10 | V2 = 7 | V2 = 9 | V2 = 13 | V2 = 22 | V2 = 28 |

Die Emulsionen werden hergestellt, indem die Komponenten von Phase B unter Rühren zu den Komponenten von Phase A gegeben werden und anschließend 1 min. mit einem Hand-Rotor-Stator-Mixer homogenisiert werden.

Im Fall der Emulsion 7 müssen die Komponenten der Phase A auf ca. 80 °C erhitzt werden, damit die wachsartigen Komponenten (Microcrystalline Wax und Castor Wax) geschmolzen werden. Man kann die Ölphase anschließend wieder auf Raumtemperatur abkühlen und sonst wie oben beschrieben verfahren.

Bei den Emulsionen 8 und 9 handelt es sich um Vergleichsemulsionen. Beim direkten Vergleich von Emulsion 2 mit Vergleichsemulsion 8 bzw. von Emulsion 3 mit Vergleichsemulsion 9 wird ersichtlich, dass mit den erfindungsgemäßen Verbindungen als Emulgatoren eine deutlich niedrigere Emulsionsviskosität erzielt wird: 6 zu 36 und 10 zu 52 Pas, die auch - im Gegensatz zu den Vergleichsemulsionen - über die Lagerzeit von mind. 2 Monaten stabil ist, während im Fall der Vergleichsemulsion 8 die Viskosität deutlich von 36 auf 22 und im Fall der Vergleichsemulsion 9 von 52 auf 28 Pas abfällt.

Die Emulsionen 4 bis 7 zeigen ferner, dass die erfindungsgemäßen Verbindungen auch in Kombination mit herkömmlichen Emulgatoren zu den gewünscht niedrigen Emulsionsviskositäten führen.

## Patentansprüche

1. Organopolysiloxan-Copolymere, enthaltend im Mittel mindestens eine über einen Spacer an das Siloxan gebundene Polyestergruppe und im Mittel mindestens eine über einen Spacer an das Siloxan gebundene hydrophile Gruppe, der allgemeinen Formel (I): 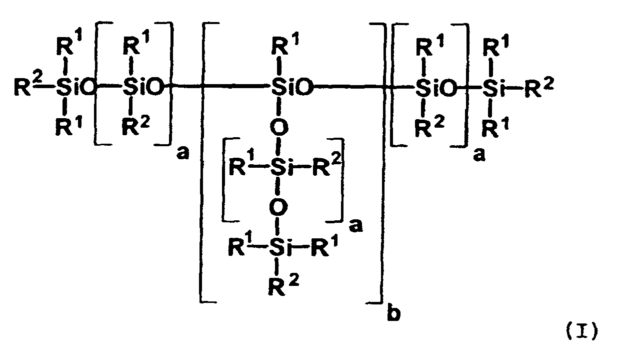
worin bedeuten
R¹ gleich oder verschieden sind und Alkylreste mit 1 bis 30 Kohlenstoffatomen oder Phenylreste,
R² unabhängig voneinander R¹, -A-R³ oder -B-R⁴ in denen
-A- eine divalente Alkylenoxy-Gruppe mit 3 bis 24 C-Atomen, die gegebenenfalls verzweigt ist und/oder Doppelbindungen enthalten kann, und/oder eine divalente Polyoxyalkylen-Gruppe der allgemeinen durchschnittlichen Formel
-R⁵-(C₂H₄O)_{q}-(C₃H₆O)ᵣ-(C₄H₈O)ₛ-
in der
q = 1 bis 100,
r = 0 bis 100,
s = 0 bis 100,
R⁵ eine divalente Alkylenoxy-Gruppe ist, mit 1 bis 24 C-Atomen, die gegebenenfalls verzweigt ist und/oder Doppelbindungen enthalten kann,
R³ ein Polyesterrest der allgemeinen Formel 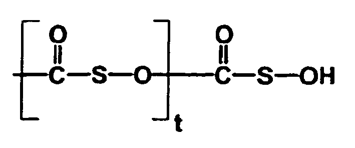 worin
t ganze Zahlen im Bereich von 1 bis 10 darstellt, und [-(O=C)-S-O-] das Fragment einer entsprechenden Hydroxycarbonsäure HO-(O=C)-S-OH bedeutet, in dem
-S- ein gegebenenfalls verzweigter und/oder Doppelbindungen enthaltender Alkylenrest mit 5 bis 30 Kohlenstoffatomen ist, mit der Maßgabe, dass zwischen der Carboxylgruppe [HO-C(O)-] und der Hydroxylgruppe[-OH] mindestens 5 Kohlenstoffatome sind;
-B- als Spacer zwischen Siloxangerüst und dem Rest R⁴ fungiert und von aus dem Stand der Technik für hydrophilmodifizierte Siloxane bekannter Art ist
R⁴ ein hydrophiler Rest der allgemeinen durchschnittlichen Formel
-R⁶-(C₂H₄O)_{q}-(C₃H₆O)ᵣ-(C₄H₈O)ₛ-R⁷
in der
q = 1 bis 100,
r = 0 bis 100,
s = 0 bis 100,
R⁶ eine divalente Alkylen- oder Alkylenoxy-Gruppe mit 1 bis 24 C-Atomen, die gegebenenfalls verzweigt ist und/oder Doppelbindungen enthalten kann;
R⁷ ein Wasserstoffatom, Alkyl- oder Acyl-Rest mit 1 bis 20 C-Atomen ist, oder
R⁴ ein Polyhydroxyorganyl-Rest, insbesondere ein Glycerin-, Polyglycerin-, Zucker- oder Zuckerderivat-Rest, ein Polyvinylalkoholrest, ein Carboxylat-, Sulfat- oder Phosphat-Rest, ein Ammonium-Rest oder ein amphoteres Betain- oder Amphoglycinat-Rest,
a einen Wert von 1 bis 1.000 und
b einen Wert von 0 bis 10 hat
mit der Maßgabe, dass im statistischen Mittel mindestens jeweils ein Rest R² = -A-R³ und R² = -B-R⁴ enthalten ist, oder für den Fall, dass kein Rest -B-R⁴ enthalten ist, mindestens ein Rest R² = -A-R³ enthalten ist, bei dem -A- einer divalenten Polyoxyalkylen-Gruppe der oben beschriebenen allgemeinen durchschnittlichen Formel
-R⁵-(C₂H₄O)_{q}-(C₃H₆O)ᵣ-(C₄H₈O)ₛ- entspricht.

2. Organopolysiloxan-Copolymere gemäß Anspruch 1, **dadurch gekennzeichnet, dass** das Fragment [-(O=C)-S-O-]ₜ dem Rest der 12-Hydroxystearinsäure oder der Ricinolsäure entspricht und t zwischen 2 und 5 liegt.

3. Organopolysiloxan-Copolymere gemäß Anspruch 1, **dadurch gekennzeichnet, dass** der hydrophile Rest R⁴ ein Rest aus der Gruppe Polyether, Polyglycerin, Polyvinylalkohol, Zucker- oder Zuckerderivate ist.

4. Organopolysiloxan-Copolymere gemäß einem der Ansprüche 1 bis 3, **dadurch gekennzeichnet, dass** b = 0 und a = 10 bis 150 ist.

5. Verfahren zur Herstellung der Verbindungen der allgemeinen Formel (I), **dadurch** gekennzeichntet, dass die Polyesterreste entweder durch Hydrosilylierung von Doppelbindungen tragenden Polyestern an Polyhydrogensiloxane oder durch Veresterung von OH-funktionellen Polysiloxanen mit freien Carboxylgruppen tragenden Polyestern und die hydrophilen Reste durch aus dem Stand der Technik bekannte Verfahren an das Polysiloxan angelagert werden.

6. Verwendung der Polysiloxan-Copolymeren der allgemeinen Formel (I) als Emulgatoren und Dispergierhilfsmittel.

7. Verwendung der Polysiloxan-Copolymeren der allgemeinen Formel (I) als Emulgatoren, gegebenenfalls unter Mitverwendung weiterer Emulgatoren, für die Herstellung niedrigviskoser W/O-Emulsionen mit hohem Gehalt an disperser Phase.

8. Dispersionen und Emulsionen, enthaltend mindestens eine der Verbindungen der allgemeinen Formel (I).

9. Kosmetische W/O-Emulsionen, enthaltend 0,5 bis 4 Gew.-%, bezogen auf die Gesamtformulierung, mindestens einer der Verbindungen der allgemeinen Formel (I).

## Claims

1. Organopolysiloxane copolymers comprising, on average, at least one polyester group bonded to the siloxane via a spacer and, on average, at least one hydrophilic group bonded to the siloxane via a spacer, of the general formula (I): 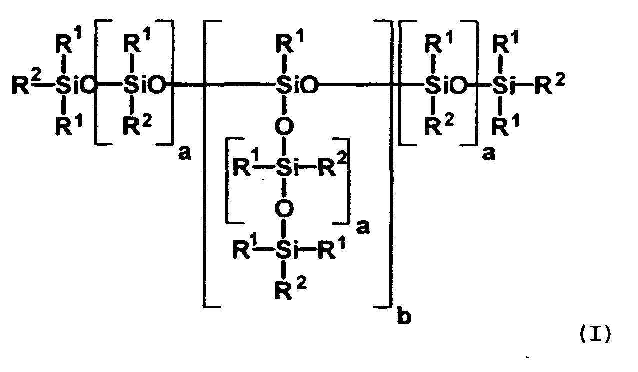
in which
R¹ are identical or different and are alkyl radicals having 1 to 30 carbon atoms or phenyl radicals,
R² independently of one another are R¹, -A-R³ or -B-R⁴ in which
-A- is a divalent alkyleneoxy group having 3 to 24 carbon atoms which is optionally branched and/or can contain double bonds,
and/or is a divalent polyoxyalkylene group of the general average formula
-R⁵-(C₂H₄O)_{q}-(C₃H₆O)ᵣ-(C₄H₈O)ₛ-
in which
q = 1 to 100,
r = 0 to 100,
s = 0 to 100,
R⁵ is a divalent alkyleneoxy group having 1 to 24 carbon atoms which is optionally branched and/or can contain double bonds,
R³ is a polyester radical of the general formula 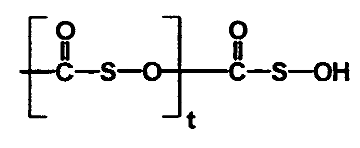 in which
t is integers in the range from 1 to 10, and [-(O=C)-S-O-] is the fragment of a corresponding hydroxycarboxylic acid HO-(O=C)-S-OH, in which
-S- is an optionally branched and/or double-bond-containing alkylene radical having 5 to 30 carbon atoms, with the proviso that at least 5 carbon atoms are between the carboxyl group [HO-C(O)-] and the hydroxyl group [-OH];
-B- acts as a spacer between siloxane backbone and the radical R⁴ and is of a type known from the prior art for hydrophilically modified siloxanes
R⁴ is a hydrophilic radical of the general average formula
-R⁶-(C₂H₄O)_{q}-(C₃H₆O)ᵣ-(C₄H₈O)ₛ-R⁷
in which
q = 1 to 100,
r = 0 to 100,
s = 0 to 100,
R⁶ is a divalent alkylene or alkyleneoxy group having 1 to 24 carbon atoms which is optionally branched and/or can contain double bonds;
R⁷ is a hydrogen atom, alkyl or acyl radical having 1 to 20 carbon atoms, or
R⁴ is a polyhydroxyorganyl radical, in particular a glycerol, polyglycerol, sugar or sugar derivative radical, a polyvinyl alcohol radical, a carboxylate, sulphate or phosphate radical, an ammonium radical or an amphoteric betaine or amphoglycinate radical,
a has a value from 1 to 1000 and
b has a value from 0 to 10
with the proviso that, on statistical average, at least in each case one radical R² = -A-R³ and R² = -B-R⁴ is present, or in the case where no radical -B-R⁴ is present, at least one radical R² = -A-R³ is present in which -A- is a divalent polyoxyalkylene group of the above-described general average formula
-R⁵-(C₂H₄O)_{q}-(C₃H₆O)ᵣ-(C₄H₈O)ₛ-.

2. Organopolysiloxane copolymers according to Claim 1, **characterized in that** the fragment [-(O=C)-S-O-]ₜ corresponds to the radical of 12-hydroxystearic acid or of ricinoleic acid and t is between 2 and 5.

3. Organopolysiloxane copolymers according to Claim 1, **characterized in that** the hydrophilic radical R⁴ is a radical from the group consisting of polyethers, polyglycerol, polyvinyl alcohol, sugar or sugar derivatives.

4. Organopolysiloxane copolymers according to any of Claims 1 to 3, **characterized in that** b = 0 and a = 10 to 150.

5. Process for the preparation of the compounds of the general formula (I), **characterized in that** it comprises adding on the polyester radicals either by hydrosilylation of polyesters carrying double bonds to polyhydrogensiloxanes, or by esterification of OH-functional polysiloxanes with polyesters carrying free carboxyl groups, and the hydrophilic radicals by processes known from the prior art to the polysiloxane.

6. Use of the polysiloxane copolymers of the general formula (I) as emulsifiers and dispersing agents.

7. Use of the polysiloxane copolymers of the general formula (I) as emulsifiers, optionally with co-use of further emulsifiers, for the preparation of low-viscosity W/O emulsions with a high content of disperse phase.

8. Dispersions and emulsions comprising at least one of the compounds of the general formula (I).

9. Cosmetic W/O emulsions comprising 0.5 to 4% by weight, based on the total formulation, of at least one of the compounds of the general formula (I).

## Revendications

1. Copolymères d'organopolysiloxane, contenant en moyenne au moins un groupe polyester lié via un écarteur au siloxane et en moyenne au moins un groupe hydrophile lié via un écarteur au siloxane, de formule générale (I): 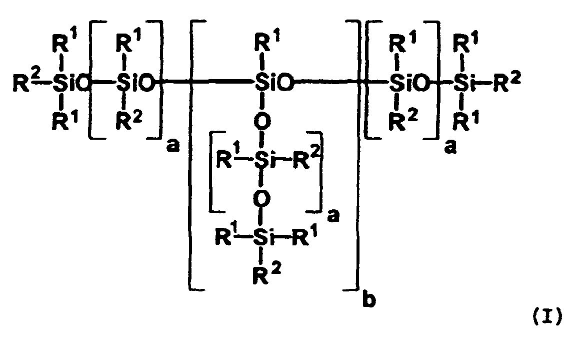
dans laquelle les groupes
R¹ sont identiques ou différents et représentent des radicaux alkyle comprenant 1 à 30 atomes de carbone ou des radicaux phényle,
R² représentent indépendamment l'un de l'autre R¹, -A- R³ ou -B-R⁴
où
-A- représente un groupe alkylénoxy comprenant 3 à 24 atomes de carbone, qui est le cas échéant ramifié et/ou qui peut contenir des doubles liaisons,
et/ou un groupe polyoxyalkylène divalent de formule générale moyenne
-R⁵-(C₂H₄O)_{q}-(C₃H₆O)ᵣ-(C₄H₈O)ₛ-
dans laquelle
q = 1 à 100,
r = 0 à 100,
s = 0 à 100,
R⁵ représente un groupe alkylénoxy divalent, comprenant 1 à 24 atomes de carbone, qui est le cas échéant ramifié et/ou qui peut contenir des doubles liaisons,
R³ représente un radical polyester de formule générale 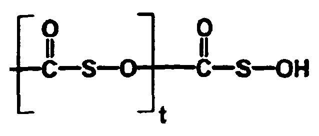 dans laquelle
t représente des nombres entiers dans la plage de 1 à 10, et [-(O=C)-S-O-] signifie le fragment d'un acide hydroxycarboxylique correspondant HO-(O=C)-S-OH, dans lequel
-S- représente un radical alkylène le cas échéant ramifié et/ou contenant des doubles liaisons comprenant 5 à 30 atomes de carbone, à condition qu'il y ait au moins 5 atomes de carbone entre le groupe carboxyle [HO-C(O-] et le groupe hydroxyle [-OH] ;
-B- agit en tant qu'écarteur entre la structure siloxane et le radical R⁴ et est du type connu par l'état de la technique pour des siloxanes modifiés de manière hydrophile,
R⁴ représente un radical hydrophile de formule générale moyenne
-R⁶-(C₂H₄O)_{q}-(C₃H₆O)ᵣ-(C₄H₈O)ₛ-R⁷
dans laquelle
q = 1 à 100,
r = 0 à 100,
s = 0 à 100,
R⁶ représente un groupe alkylène ou alkylénoxy divalent comprenant 1 à 24 atomes de carbone, qui est le cas échéant ramifié et/ou qui peut contenir des doubles liaisons;
R⁷ représente un atome d'hydrogène, un radical alkyle ou acyle comprenant 1 à 20 atomes de carbone, ou
R⁴ représente un radical polyhydroxyorganyle, en particulier un radical glycérol, polyglycérol, de sucre ou d'un dérivé de sucre, un radical poly(alcool vinylique), un radical carboxylate, sulfate ou phosphate, un radical ammonium ou un radical bétaïne amphotère ou un radical amphoglycinate,
a vaut 1 à 1000 et
b vaut 0 à 10
à condition qu'en moyenne statistique, au moins à chaque fois un radical R² = -A-R³ et R² = -B-R⁴ soient contenus ou, dans le cas où il n'y aurait pas de radical -B-R⁴, au moins un radical R² = -A- R³ soit contenu, dans lequel -A- correspond à un groupe polyoxyalkylène divalent de formule générale moyenne décrite ci-dessus
-R⁵-(C₂H₄O)_{q}-(C₃H₅O)ᵣ-(C₄H₈O)ₛ-.

2. Copolymères d'organopolysiloxane selon la revendication 1, **caractérisés en ce que** le fragment [-(O=C)-S-O-]ₜ correspond au radical de l'acide 12-hydroxystéarique ou de l'acide ricinoléique et t est situé entre 2 et 5.

3. Copolymères d'organopolysiloxane selon la revendication 1, **caractérisés en ce que** le radical hydrophile R⁴ est un radical du groupe des polyéthers, des polyglycérols, des poly(alcools vinyliques), des sucres ou des dérivés du sucre.

4. Copolymères d'organopolysiloxane selon l'une quelconque des revendications 1 à 3, **caractérisés en ce que** b = 0 et a = 10 à 150.

5. Procédé pour la préparation des composés de formule générale (I), **caractérisé en ce que** les radicaux polyester sont additionnés par hydrosilylation de polyesters portant des doubles liaisons sur des polyhydrogénosiloxanes ou par estérification de polysiloxanes à fonctionnalité OH avec des polyesters portant des groupes carboxyle libres et les radicaux hydrophiles sont additionnés sur le polysiloxane par des procédés connus par l'état de la technique.

6. Utilisation des copolymères de polysiloxane de formule générale (I) comme émulsifiants et adjuvants de dispersion.

7. Utilisation des copolymères de polysiloxane de formule générale (I) comme émulsifiants, le cas échéant avec une utilisation conjointe d'autres émulsifiants pour la préparation d'émulsions E/H de basse viscosité présentant une teneur élevée en phase dispersée.

8. Dispersions et émulsions contenant au moins un des composés de formule générale (I).

9. Emulsions E/H cosmétiques, contenant 0,5 à 4% en poids, par rapport à la formulation totale, d'au moins un des composés de formule générale (I).
